Europäisches Patentamt

European Patent Office

Office européen des brevets

(11) Veröffentlichungsnummer : **0 052 133**
**B1**

## (12) EUROPÄISCHE PATENTSCHRIFT

(45) Veröffentlichungstag der Patentschrift :
26.09.84

(21) Anmeldenummer : **81901525.6**

(22) Anmeldetag : **26.05.81**

(86) Internationale Anmeldenummer :
**PCT/DE 81/00076**

(87) Internationale Veröffentlichungsnummer :
**WO/8103418 (10.12.81 Gazette 81/29)**

(51) Int. Cl.³ : **A 61 B   5/00, G 01 J   5/00**

(54) VERFAHREN ZU ERFASSUNG UND DARSTELLUNG THERMOGRAPHISCHER BILDER.

(30) Priorität : **29.05.80 DE 3020359**

(43) Veröffentlichungstag der Anmeldung :
**26.05.82 Patentblatt 82/21**

(45) Bekanntmachung des Hinweises auf die Patenterteilung : **26.09.84 Patentblatt 84/39**

(84) Benannte Vertragsstaaten :
**CH DE FR GB LI NL SE**

(56) Entgegenhaltungen :
**DE-B- 1 284 659**
**DE-B- 2 835 602**
**FR-A- 2 142 117**
**US-A- 4 186 748**
**US-A- 4 192 004**
**IEEE Conference Publication on Low Light and Thermal Imaging Systems, nr. 124, 3-5 märz 1975 (London, GB), J.R. DAVY: "Medical applications thermography", Seiten 171-179**
**IEEE MTT-S International Microwave Symposium Digest, 1979 (New York, US), J.E. Thompson et al. "Tumor detection using microwave enhanced thermography and computer aided image analysis", Seiten 39-44**

(73) Patentinhaber : **FA. CARL ZEISS; CARL ZEISS-STIFTUNG**
**Carl-Zeiss-Strasse 4-54**
**D-7082 Oberkochen (DE)**

(72) Erfinder : **WOLLNIK, Hermann**
**Auf der Platte 30**
**D-6301 Fernwald 2 (DE)**
Erfinder : **HAAS, Rüdiger**
**Kollenbergstrasse 24**
**D-6331 Altenkirchen (DE)**
Erfinder : **KASSEN, Folkert**
**Lärchenweg 13**
**D-6301 Lahnau-Atzbach (DE)**

## Beschreibung

Die Erfindung betrifft ein Verfahren zur Erfassung und Darstellung thermographischer Bilder von biologischen oder technischen Strukturen, bei dem die Temperaturwerte an jedem Bildpunkt eines Thermogramms digital erfaßt, ausgewertet und die Auswertungsergebnisse dargestellt werden. Ein derartiges Verfahren ist z. B. aus dem Dokument IEEE MTT-S International Microwave Symposium Digest, 1979 (New York), S. 39-44 bekannt, bei dem die Temperaturwertverteilung in einem computererrechneten X,Y,T-Diagramm dargestellt wird.

Thermogramme oder thermographische Bilder können für die verschiedensten Zwecke eingesetzt werden, um die Temperaturverteilungen bei biologischen oder technischen Strukturen zu erfassen und zu untersuchen. Dabei ist es in vielen Fällen schwierig, etwa bei einem durch Infrarotfotographie erzeugten Bild lokalbegrenzte unauffällige Temperaturverteilungen einerseits und lokalbegrenzte « anormale » Temperaturen andererseits zu unterscheiden.

Seit Jahrzehnten ist in der Fachwelt bekannt, daß Tumore (Karzinome) im menschlichen Körper ein « anormales », von der Körperschale abweichendes Temperaturverhalten haben. Um Tumore mit Hilfe der Thermographie festzustellen, ist bisher so vorgegangen worden, daß mit einer Thermokamera von dem zu überprüfenden Bereich thermographische Bilder angefertigt und diese — ggfs. unter Zwischenschaltung eines Analog-Digital-Wandlers — auf einem Bildschirm zur Anzeige gebracht werden. Um dabei Tumore besser identifizieren zu können, wird die Körpertemperatur durch Abkühlung herabgesetzt. Der erkrankte Bereich des Körpers folgt dieser Temperaturänderung nur begrenzt, so daß aus diesem lokal unterschiedlichen Temperaturverhalten Rückschlüsse auf evtl. vorhandene Tumore gezogen wurden. Dieses Verfahren ist vor allem bei der Ferststellung von Mammakarzinomen eingesetzt worden. Die Auswertung der Bildanzeige erfolgte dabei ausschließlich visuell, d. h. man hat versucht anhand unterschiedlicher Grauton- bzw. Farbwerte des thermographischen Bildes Karzinome zu identifizieren.

Wegen der bei dieser Auswertung gegebenen erheblichen Unsicherheit konnte sich die Thermographie in der Praxis bei der Feststellung von Krankheitsherden in der menschlichen Körperschale nicht durchsetzen.

Aufgabe der Erfindung ist es, ein Verfahren der eingangs genannten Art vorzuschlagen, mit dem es möglich ist, anormale Temperaturverteilungen auch dann zu erkennen, wenn diese sehr klein sind oder die entsprechenden Bereiche sich nur geringfügig hinsichtlich ihrer Temperaturwertes von den benachbarten Temperaturwerten unterscheiden.

Die Lösung dieser Aufgabe erfolgt gemäß der Erfindung dadurch, daß die Steilheit der lokalen Temperaturänderung, d. h. der Wert des Temperaturgradienten für jeden Bildpunkt vorzugsweise aus seiner Temperatur und den gemessenen Temperaturwerten, seiner Nachbarpunkte, errechnet und die örtliche Verteilung der Temperaturgradientenwerte für einen ausgewählten Bildbereich dargestellt wird.

Die Ermittlung der Temperaturgradientenwerte für jeden Bildpunkt erfolgt beispielsweise durch einen Prozeßrechner, dessen Ausgangssignale als Bild oder Bilder dargestellt werden.

Eine weitere Möglichkeit zur Ermittlung der Temperaturgradienten für jeden Bildpunkt besteht darin die thermographischen Signale einer Fourier-Analyse zu unterwerfen und nur hohe Ortsfrequenzen auszufiltern und gegebenenfalls darzustellen.

Prinzipiell ist es ohne Belang nach welchem Algorithmus die Temperatur-gradienten ermittelt werden, da die heute verfügbaren Rechner in jedem Falle die gesuchten Werte in kürzester Zeit errechnen können.

Kleinere Temperaturänderungen, die bereits beim Entstehen von gefährlichen Situationen, vor allem im biologisch-medizinischen Bereich auftreten lassen sich aufgrund ihrer Unauffälligkeit in einer bildlichen Darstellung der Temperaturwerte nur sehr schwer erkennen. Weitaus signifikanter ist hier die Darstellung der Temperaturgradienten für jeden Bildpunkt oder für einen ausgewählten Bereich.

Besonders vorteilhaft ist es dabei für die Werte der Temperaturgradienten gestaffelte Schwellwerte vorzugeben und den sich so ergebenden Wertbereichen Farbstufen zuzuordnen. Die Werte der Temperaturgradienten treten dann in der bildlichen Darstellung mit unterschiedlichen Farben in Erscheinung, so daß es möglich wird auf den ersten Blick Gefahrenbereiche zu erkennen.

Es ist auch möglich den Temperaturgradienten-Werten einen vorwählbaren Schwellwert zuzuordnen, der beispielsweise den pathologischen Bereich abgrenzt und die unter- und oberhalb dieses Schwellwertes liegenden Meßwerte in schwarz/weiß-Darstellung oder einer anderen einfarbigen Darstellung wiederzugeben.

Die Erfindung kann in verschiedenen Bereichen der Naturwissenschaft und Technik, vor allem im Bereich der Medizin, zum Einsatz kommen. Im medizinischen Bereich wird mittels des Verfahrens nach der Erfindung die Feststellung von Karzinomen, vor allem Mammakarzinomen, mit Hilfe der Thermographie praktikabel.

Darüber hinaus kann die Erfindung für andere medizinische Untersuchungen, z. B. für die Feststellung von Entzündungen, rheumatischen Erkrankungen in Gelenken usw. zum Einsatz kommen. Im biologischen Bereich können Luftaufnahmen von Wäldern angefertigt werden, um deren Wasserhaushalt zu untersuchen. Die thermographischen Aufnahmen werden dabei nach dem Verfahren der Erfindung ausgewertet, um exakte Angaben über die Wasserverteilung zu erzielen.

Im technischen Bereich kann die Erfindung bei der Überprüfung der Wärmedämmung von Gebäuden auf der Grundlage thermographischer Aufnahmen zum Einsatz kommen sowie für die thermographische Untersuchung von metallischen Werkstücken.

Grundsätzlich kann das erfindungsgemäße Verfahren überall dann zum Einsatz gelangen, wenn zusätzliche Informationen über einen exakten Temperaturverlauf erforderlich sind, welche sich aus dem thermographischen Bild der absoluen Temperaturwerte nich oder nur schwer entnehmen lassen.

Das Verfahren nach der Erfindung läßt sich auch so ausgestalten, daß Temperaturgradientenfelder für unterschiedliche Temperaturen technischer Strukturen aufgenommen und miteinander verglichen werden. Dieser Vergleich kann durch Bildung von Differenzen, Quotienten oder anderen mathematischen Relationen, wie etwa die Abweichung von linearen und/oder quadratischen Extrapolationen sowie solcher höherer Ordnung erfolgen.

Zugleich mit der Aufnahme der Temperaturgradientenfelder können auch die Temperaturfelder bei verschiedenen Temperaturen technischer Strukturen ausgewertet werden, indem z. B. für jeden Bildpunkt des untersuchten Bereiches Differenzen, Quotienten oder andere mathematische Relationen zwischen den verschiedenen Temperaturfeldern ermittelt und neben den Temperaturgradientenfeldern dargestellt oder diesen überlagert werden.

Die Darstellung der Temperatur- und Temperaturgradientenfelder nebeneinander kann auf zwei verschiedenen Bildschirmen oder auf einem gemeinsamen, etwa mittig geteilten Bildschirm erfolgen. Zur überlagerten Darstellung der erwähnten Felder ist es zweckmäßig diese auf einem gemeinsamen Bildschirm darzustellen, wobei eine Größe, beispielsweise das Temperaturfeld schwarz/weiß und das andere Feld entweder einfarbig mit abgestufter Farbsättigung oder in verschiedenen Farben wiedergegeben wird. Wählt man nur einen, einen vorgegebenen Bereich abgrenzenden Schwellwert vor, so kann die Darstellung so erfolgen, daß im Bild nur dieser Bereich wiedergegeben wird.

Vorteilhaft kann es auch sein beide Felder farbig darzustellen, wobei die Farben so gewählt sind, daß die zu identifizierenden Bereiche durch Mischung eine besonders auffällige Farbe erhalten.

Um eine besonders einfache Auswertung zu gewährleisten ist es vorteilhaft die Darstellung des Temperaturgradientenfeldes oder auch die überlagerte Darstellung dieses Feldes mit dem Temperaturfeld so zu gestalten, daß die zu identifizierenden Bereiche, Beispielsweise Bereiche mit einem großen Temperaturgradienten blinken.

Die Darstellung des Ergebnisses der Auswertung thermographischer Bilder kann auch ohne Einsatz von Von Bildschirmen erfolgen, z. B. durch Aufzeichnen von graphischen Darstellungen oder durch tabellarisch erfaßte Zahlenwerte.

Bei dem Verfahren nach der Erfindung werden die Temperaturgradienten für jeden Bildpunkt rechnerisch ermittelt. Die dabei gewonnenen Signale werden in einer vorteilhaften Weiterbildung der Erfindung zur automatischen Scharfeinstellung der thermographischen Kamera verwendet. Dazu kann beispielsweise unter Bildung der Summe der Gradientensignale eines mehr oder weniger großen Bildbereiches die Fokussierung der Kamera automatisch solange verändert werden bis diese Summe ihr Maximum erreicht. Es ist auch möglich zur automatischen Scharfeinstellung nur die hohen Frequenzen im Gradienten-Signalfeld zu verwenden.

Die Erfindung wird nachstehend anhand der Figuren 1 bis 5 der beigefügten Zeichnungen näher erläutert. Im einzelnen zeigen :

Figur 1 ein schematisches Blockschaltbild eines Ausführungsbeispiels einer Anordnung zur Auswertung thermographischer Bilder nach dem erfindungsgemäßen Verfahren ;

Figur 2 den Temperaturverlauf längs einer Zeile eines Rasters des untersuchten Bereichs ;

Figur 3 eine Darstellung der Kurve gemäß Fig. 2 durch unterschiedliche farben bzw. unterschiedliche Grautöne ;

Figur 4 eine Kurve, welche den aus der Kurve der Fig. 2 ermittelten Temperaturgradienten in seinem Verlauf längs der Rasterzeile zeigt ;

Figur 5 eine Darstellung der Kurve gemäß Fig. 4 durch unterschiedliche Farben bzw. unterschiedliche Grautöne.

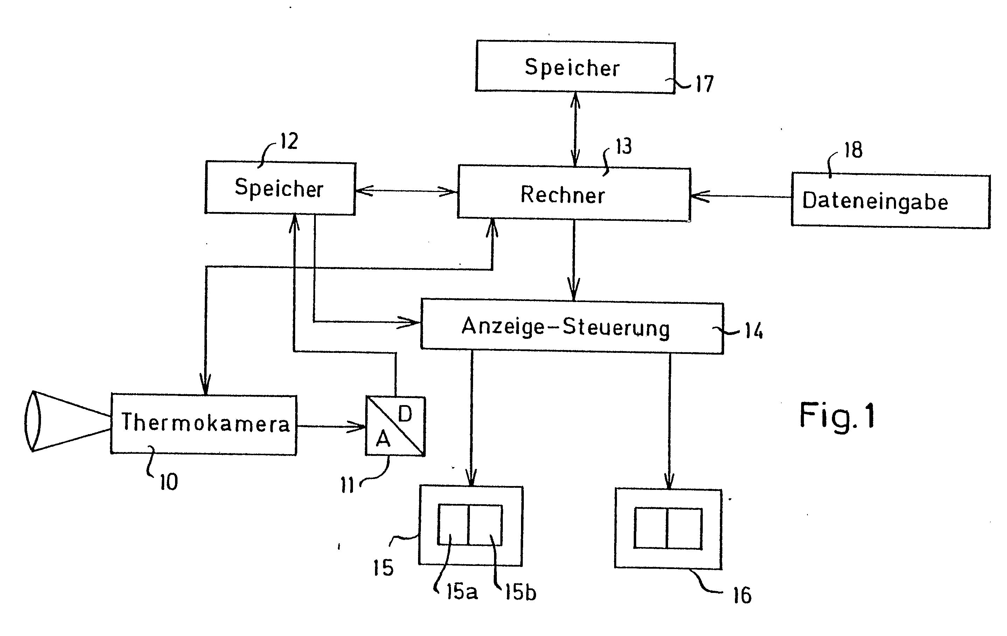

Bei der in Fig. 1 dargestellten Anordnung ist mit 10 eine Thermokamera zur Aufnahme thermographischer Bildern z. B. eines Bereichs eines menschlichen Körpers bezeichnet. Die Signal der Thermokamera 10 werden einem an diese angeschlossenen Analog-Digital-Wandler 11 zugeführt. An diesem wiederum ist ein erster Speicher 12 zur Aufnahme der digitalisierten Bildsignale angeschlossen.

Bei dem Verfahren nach der Erfindung werden die Temperaturgradienten für jeden Bildpunkt rechnerisch ermittelt. Die dabei gewonnenen Signale werden in einer vorteilhaften Weiterbildung der Erfindung zur automatischen Scharfeinstellung der thermographischen Kamera verwendet. Dazu kann beispielsweise unter Bildung der Summe der Gradientensignale eines mehr oder weniger großen Bildbereiches die Fokussierung der Kamera automatisch solange verändert werden bis diese Summe ihr Maximum erreicht. Es ist auch möglich zur automatischen Scharfeinstellung nur die hohen Frequenzen im Gradienten-Signalfeld zu verwenden.

Der Speicher 12 ist seinerseits mit einem beispielsweise als Prozeßrechner ausgebildeten Rechner 13 verbunden, um einerseits einen Datenaustausch und andererseits die Rückmeldung von Signalen an den Speicher 12 zu ermöglichen wenn ein Rechenkomplex im Rechner 13 abgeschlossen ist.

Der Speicher 12 und der Rechner 13 sind jeweils an eine Anzeigesteuerung 14 angeschlossen, die ihrerseits mit Monitoren 15 und 16 verbunden ist, um die jeweiligen Temperaturgradientenwerte, Temperaturwerte oder aber Differenzwerte bzw. Quotientenwerte aus Temperaturgradienten bzw. Temperaturen zur Anzeige zu bringen.

Ferner ist der Rechner 13 zum gegenseitigen Datenaustausch mit einem zweiten Speicher 17 oder einem weiteren Rechner mit angeschlossenem Speicher verbunden, der zur langfristigen Speicherung von Daten vorgesehen ist und seine Daten vom Rechner 13 erhält. Diesem werden weitere Daten über eine Dateneingabe 18 etwa einem weiteren Rechner zugeführt, z. B. Steuersignale für Start, Stop usw.

Das zu untersuchende Objekt wird mit dem Thermokamera 10 aufgenommen, die gemessenen Werte im Analog-Digital-Wandler 11 digitalisiert und im Speicher 12 angespeichert. Zu diesem Zweck wird das zu untersuchende Objekt in ein Raster von z. B. 64 × 64 Bildpunkten oder 256 × 256 Bildpunkten mit einem Abstand der Bildpunkte voneinander von z. B. 5 mm bzw. 1,25 mm unterteilt. Der Rechner 13 ruft die im Speicher 12 gespeicherten Temperaturwerte ab, speichert sie zum einen im Speicher 17 (großer Langzeitspeicher) ab und verarbeitet zum anderen die abgerufenen Daten, um für jeden Bildpunkt die Temperaturgradienten zu errechnen. In Abhängigkeit von der gewünschten Berechnungsgenauigkeit können dabei die Temperaturen von jeweils vier oder acht oder mehr benachbarten Bildpunkten des Rasters bei der Berechnung berücksichtigt werden.

Die so errechneten Temperaturgradientenwerte werden im Rechner aufsummiert und mit dem Summensignal wird die Fokussierung der Kamera 10 automatisch solange geregelt bis das Summensignal sein Maximum erreicht und die Fokussierungseinstellung ihre stabile optimale Lage erreicht.

Die nach dieser Einstellung ermittelten Temperaturgradientenwerte werden im Speicher 12 gespeichert. Bei Beendigung des Berechnungsvorganges gibt der Rechner 13 eine Rückmeldung an den Speicher 12. Dieser gibt daraufhin die gespeicherten Signale der gemessenen Temperaturwerte und der errechneten Temperaturgradientenwerte zur Anzeigensteuerung 14. Diese leitet die Signale beispielsweise dem Monitor 15 zu, auf dessen Bildschirm sie sichtbar gemacht werden.

Dabei können die Temperaturwerte einerseits und die Temperaturgradientenwerte andererseits entweder auf zwei nebeneinanderliegenden Bildschirm-Bereichen 15a, 15b des Monitors 15 erscheinen oder aber überlagert dargestellt werden.

Um die jeweiligen thermographischen Bilder gut auswerten zu können, werden über die Dateneingabe 18 für die Temperaturwerte und Temperaturgradientenwerte mehr oder weniger fein gestaffelte Schwellwerte vorgegeben. Der Rechner 13 ordnet den Temperatur- und Temperaturgradientensignalen entsprechend den vorgegebenen Schwellwerten vorbestimmte Grautöne oder Farbstufen zu, die dann auf den Bildschirmen 15a, 15b oder 16 erscheinen.

Will man Temperaturwerte und Temperaturgradientenwerte nebeneinander darstellen, so kann man wahlweise Grautöne oder Farbstufen verwenden, wobei die farbige Darstellung der thermographischen Bilder in vielen Fällen vorzuziehen sein wird.

Alternativ oder zusätzlich dazu können kritische Bereiche auch dadurch sichtbar gemacht werden, daß man die jeweiligen Punkte aufblinken läßt.

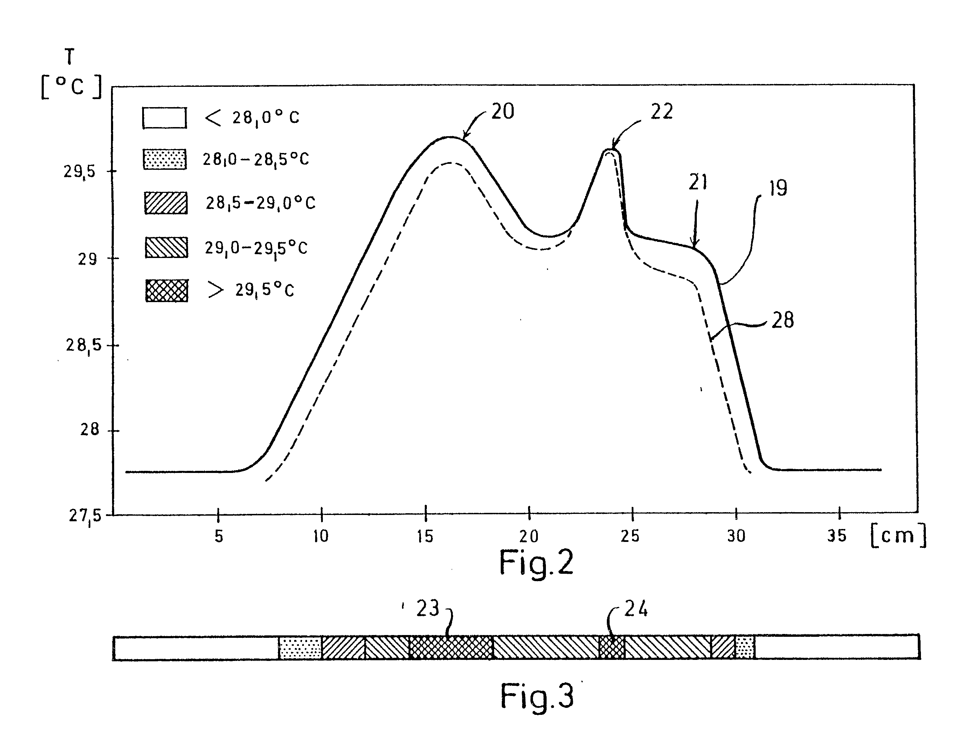

Ein Beispiel für eine optische Darstellung der ermittelten bzw. errechneten Werte ist in Fig. 2 bis 5 dargestellt. Fig. 2 zeigt in Gestalt einer Kurve 19 den Verlauf der gemessenen Temperaturwerte längs einer Zeile eines mit der kamera 10 aufgenommenen Bildes. Es kann sich dabei um eine Bildzeile eines Thermogramms handeln, das sich über einen 35 cm langen Hautstreifen erstreckt. Die gemessenen Temperaturwerte sollen im Bereich von zwei Maxima 20 und 21 der Kurve 19 Temperaturen wiedergeben, die sich beispielsweise aus hautnahen Blutgefäßen ergeben. Zwischen diesen Maxima 20 und 21 ist ein weiteres maximum 22 dargestellt, das auf einen verhältnismäßig kleinen Tumor in der Körperschale zurückzuführen sei.

In Fig. 2 ist der bei einer bestimmten mittleren Temperatur gemessenen Kurve 19 eine zweite Kurve 28 gegenübergestellt, die einer niedrigeren mittleren Körperoberflächentemperatur entspricht. Die « normal » reagierenden Bereiche der Körperschale zeigen hier durchgängig niedrigere Temperaturwerte, während im Bereich des Maximums 22 im wesentlichen die Temperaturwerte der Kurve 19 festgestellt werden.

Eine Darstellung der Farb- bzw. Grautonwerte zu den Temperaturbereichen der voranstehend erläuterten Kurve 19 ergibt laut Fig. 3, daß Farbbereiche 23, 24 (bzw. Grautonbereiche) für die Maxima 20 und 22 übereinstimmen, obwohl dieser Kurvenbildung unterschiedliche, zu identifizierende Ursachen zugrundeliegen.

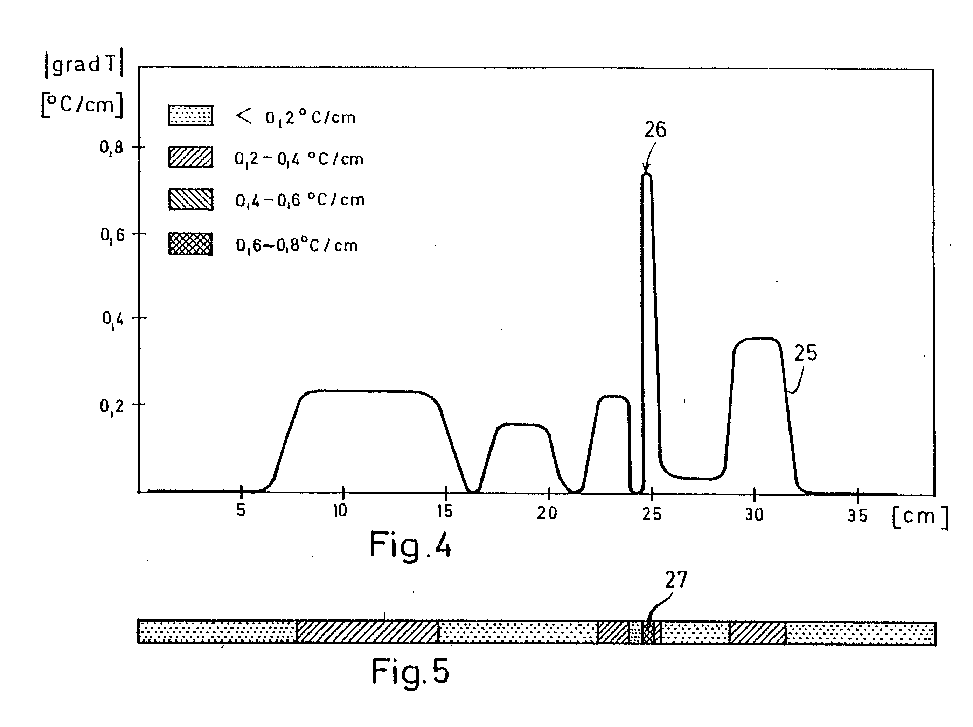

Fig. 4 zeigt eine Kurve 25, die die zu den Temperaturwerten der Kurve 19 der Fig. 2 gehörenden Temperaturgradientenwerte wiedergibt. Wie aus der Kurve 25 ersichtlich, ist ein Maximum 26, welches dem Abfall der Kurve 19 nach dem maximum 22 zuzuordnen ist, gegenüber dem übrigen Verlauf der Kurve 25 deutlich abgesetzt.

Wird für die Kurve 25 eine bildliche Darstellung entsprechend Fig. 3 aufgezeichnet, also mit den vorgegebenen Schwellwertbereichen zugeordneten Farbbereichen, so ergibt sich, daß ein Farbbereich 27 deutlich gegenüber anderen, auch untereinander verschiedenen Farbbereichen abgesetzt ist.

Bei einem alternativen Verfahren zur Auswertung thermographischer Aufnahmen werden Temperaturwerte bei wiederholten Aufnahmen bzw. Messungen, jedoch unterschiedlichen mittleren Temperaturen technischer Strukturen einander gegenübergestellt.

Mit Hilfe der Anordnung nach Fig. 1 werden die Temperaturwerte verschiedener Messungen bzw. Aufnahmen technischer Strukturen einander rechnerisch gegenübergestellt, indem für jeden Bildpunkt beispielsweise die Differenzen oder Quotientenwerte ermittelt und auf einem Bildschirm, z. B. des Monitors 16, dargestellt werden. Es kann dabei so vorgegangen werden, daß die Werte eines zuvor bei einer anderen mittleren Strukturtemperatur aufgenommenen Thermogramms aus dem Speicher 17 abgerufen und im Rechner 13 den Werten des neu aufgenommenen Thermogramms gegenübergestellt werden.

Dieses Verfahren kann mit der Ermittlung und Darstellung der Temperaturgradientenwerte gleichzeitig durchgeführt werden, wobei die letztgenannten Daten gleichzeitig mittels des anderen Monitors 15 dargestellt sind. Es können dadurch auch Bilder einander gegenübergestellt werden, die mit größerem zeitlichen Abstand voneinander aufgenommen wurden. Man kann dadurch sowohl lokale als auch zeitliche Temperaturänderungen deutlich machen. Der als Langzeitspeicher ausgebeildete Speicher 17 nimmt hierfür Daten auf entsprechenden Datenträgern für die Speicherung über Jahre hinweg auf.

## Ansprüche

1. Verfahren zur Erfassung und Darstellung thermographischer Bilder von biologischen oder technischen Strukturen, wobei die Temperaturwerte an jedem Bildpunkt eines Thermogramms digital erfaßt, ausgewertet und die Auswertungsergebnisse dargestellt werden, dadurch gekennzeichnet, daß die Steilheit der lokalen Temperaturänderung, d. h. der Wert des Temperaturgradienten, für jeden Bildpunk vorzugsweise aus seiner Temperatur und den gemessenen Temperaturwerten seiner Nachbarpunkte, errechnet und die örtliche Verteilung der Temperaturgradientenwerte für einen ausgewählten Bildbereich dargestellt wird.

2. Verfahren nach Anspruch 1, dadurch gekennzeichnet, daß gleichzeitig mit dem Temperaturgradientenfeld das Temperaturfeld des Thermogramms dargestellt wird.

3. Verfahren nach Anspruch 1 und 2, dadurch gekennzeichnet, daß die Temperaturwerte und/oder die Temperaturgradientenwerte des Thermogramms in mehrere gestaffelte Schwellwert-Bereiche eingeordnet werden und den Werten in diesen bereichen optisch darstellbare Markierungen, insbesondere Farbstufen, zugeordnet werden.

4. Verfahren nach Anspruch 1 und 2, dadurch gekennzeichnet, daß den Temperaturwerten und/oder den Temperaturgradientenwerten ein vorwählbarer Schwellwert zugeordnet wird, und daß die unter- und oberhalb dieses Schwellwertes liegenden Meßwerte in schwarz/weiß-Darstellung wiedergegeben werden.

5. Verfahren nach Anspruch 1, dadurch gekennzeichnet, daß vor der Ermittlung der Temperaturgradienten zunächst die mittlere Temperatur in ausgewählten Bildausschnitten errechnet und dann die Temperaturgradienten für jeden Bildpunkt, bezogen auf den jeweiligen Wert dieser mittleren Temperatur, bestimmt und zur Anzeige gebracht werden.

6. Verfahren nach Anspruch 1 und 5, dadurch gekennzeichnet, daß das Temperaturgradienten-Signal zur automatischen Fokussierung des Systems zur Aufnahme der thermographischen Bilder verwendet wird.

7. Verfahren zur Erfassung und Darstellung thermographische Bilder von technischen Strukturen, wobei die Temperaturwerte an jedem Bildpunkt eines Thermogramms digital erfaßt, ausgewertet und die Auswertungsergebnisse dargestellt werden, dadurch gekennzeichnet, daß bei unterschiedlichen mittleren Temperaturen der Strukturen thermographischer Bilder aufgenommen werden, daß in jedem dieser Bilder zu jedem Bildpunkt der Wert des Temperaturgradienten errechnet wird, und daß die unterschiedlichen Strukturtemperaturen entsprechenden Temperatur- und/oder Temperaturgradientenfelder mathematisch verglichen und die Änderungen der Bilder zur Anzeige gebracht werden.

8. Verfahren nach einem der Ansprüche 1 bis 7, dadurch gekennzeichnet, daß die für ein erstes Thermogramm ermittelten Temperaturwerte und die daraus errechneten Temperaturgradientenwerte auf einem ersten Bildschirm und gleichzeitig die durch Vergleich des ersten Thermogramms mit einem zweiten Thermogramm errechneten Differenz- bzw. Quotientenwerte für die Temperaturen und/oder Temperaturgradienten auf einem zweiten Bildschirm sichtbar gemacht werden.

9. Verfahren nach einem der Ansprüche 2, 3 und 7, dadurch gekennzeichnet, daß die Temperaturwerte und die Temperaturgradientenwerte der Thermogramme in zwei nebeneinander liegenden Bereichen auf einem gemeinsamen Bildschirm zur Anzeige gebracht werden.

10. Verfahren nach einem der Ansprüche 2, 3 und 7, dadurch gekennzeichnet, daß die Temperaturwerte und die Temperaturgradientenwerte der Thermogramme übereinander auf dem gemeinsamen Bildschirm sichtbar gemacht werden, wobei das Temperaturfeld und diejenigen Orte, an denen vorgegebene Schwellwerte der Temperaturen und/oder Temperaturgradienten überschritten werden, optisch gegeneinander abgesetzt sind.

## Claims

1. Method for evaluating and displaying thermographic records of biological or technical structures, in which the temperature values at each image point of a thermogram are digitized, evaluated and the evaluation results are displayed, characterized in that the slope of the local temperature change, i. e. the temperature-gradient value for each image point is calculated preferably of its temperature and the measured temperature values of its neighboring points, and that the local distribution of the temperature-gradient values for a selected image region is displayed.

2. Method according to claim 1, characterized in that the temperature field of the thermogram is displayed simultaneously with the temperature-gradient field.

3. Method according to claim 1 and 2, characterized in that the temperature values and/or the temperature-gradient values of the thermogram are classified in several graduated threshold-value regions and that optically displayable markings, preferably color shades are associated with the values in said regions.

4. Method according to claims 1 and 2, characterised in that a preselectable threshold-value is associated with the temperature values and/or the temperature-gradient values, and that measured values lying below and above said threshold-value are reproduced in a black-and-white display.

5. Method according to claim 1, characterized in that, before the determination of the temperature-gradient values the average temperature in selected sections of the image is calculated and that then the temperature-gradient values for each image point, referred to the corresponding value of said average temperature are determined and displayed.

6. Method according to claims 1 and 5, characterized in that the temperature-gradient-signal is used for the automatic focusing of the system for recording the thermographic images.

7. Method for evaluating and displaying thermographic images of technical structures, in which the temperature values at each image point of a thermogram are digitized, evaluated and the evaluation results are displayed, characterized in that thermographic image are recorded at different average temperatures of the structures, that the temperature-gradient value for each image point is calculated in each of said images, and that temperature fields and/or temperature-gradient fields corresponding to different structure-temperatures are compared mathematically and the changes in the images are displayed.

8. Method according to one of claims 1 to 7, characterized in that the temperature values determined for a first thermogram and the temperature-gradient values calculated therefrom are displayed on a first image screen and that simultaneously the difference-values or the quotient values calculated by comparison of the first thermogram with a second thermogram are displayed on a second image screen.

9. Method according to one of claims 2, 3 and 7, characterized in that the temperature values and the temperature-gradient values of the ther-mograms are displayed in two regions located side-by-side on a common image screen.

10. Method according to one of claims 2, 3 and 7, characterized in that the temperature values and the temperature-gradient values of the ther-mograms are displayed superimposed on a common image screen, whereby the temperature field and those places where predetermined threshold-values of the temperatures and/or the temperature-gradients are exceeded, are optically distinguished from each other.

## Revendications

1. Procédé pour la saisie et la représentation d'images thermographiques de structures biologiques ou techniques, les valeurs de la température étant saisies d'une manière numérique et évaluées en chaque point image d'un thermogramme, et les résultats de l'évaluation étant représentés, caractérisé en ce qu'on calcule la pente de la variation locale de température, c'est-à-dire la valeur du gradient de température pour chaque point image, de préférence à partir de sa température et des valeurs mesurées de la température de ses points voisins, et qu'on représente la répartition locale des valeurs du gradient de température pour une zone image sélectionnée.

2. Procédé selon la revendication 1, caractérisé en ce qu'on représente simultanément au champ de gradients de températures le champ de températures du thermogramme.

3. Procédé selon l'une quelconque des revendications 1 ou 2, caractérisé en ce que les valeurs de la température et/ou les valeurs des gradients de températures du thermogramme sont ordonnées dans plusieurs domaines de seuils échelonnés, des marques optiquement représentables, en particulier des échelles de couleurs, étant attribuées aux valeurs de ces domaines.

4. Procédé selon l'une quelconque des revendications 1 ou 2, caractérisé en ce qu'un seuil présélectionnable est attribué aux valeurs de la température et/ou des gradients de températures, et que les valeurs de mesure se trouvant au-dessus ou au-dessous de ce seuil sont rendues selon une représentation en noir et blanc.

5. Procédé selon la revendication 1, caractérisé en ce que, avant la détermination des gradients de températures, on calcule tout d'abord la température moyenne sur des sections présélectionnées d'images, puisqu'on détermine et visualise les gradients de températures pour chaque point image, sur la base de la valeur, en chaque point, de cette température moyenne.

6. Procédé selon l'une des revendications 1 ou 5, caractérisé en ce que le signal gradient de températures est utilisé pour la mise au point automatique du système d'enregistrement des images thermographiques.

7. Procédé pour la saisie et la représentation d'images thermographiques de structures techniques, les valeurs de la température étant saisies et évaluées d'une manière digitale en chaque point image d'un thermogramme, et les résultats de l'évaluation étant représentés, caractérisé en ce qu'on enregistre des images thermographiques à différentes températures moyennes des structures ; que l'on calcule dans chacune de ces images, en chaque point image, la valeur du gradient de température ; et qu'on compare par un procédé mathématique les différents champs de températures et/ou de gradients de températures correspondants aux températures de la structure, et que l'on visualise les variations des images.

8. Procédé selon l'une quelconque des revendications 1 à 7, caractérisé en ce que l'on visualise sur un premier écran les valeurs de la température déterminées pour un premier thermogramme et les valeurs du gradient de température calculées à partir d'elles et, simultanément, sur un deuxième écran, les différences ou quotients, calculés par une comparaison du premier thermogramme et du deuxième thermogramme, pour ce qui est des températures et/ou des gradients de températures.

9. Procédé selon l'une des revendications 2, 3 ou 7, caractérisé en ce que les valeurs de la température et les valeurs des gradients de température du thermogramme sont affichées sur un écran commun dans deux domaines disposés l'un à côté de l'autre.

10. Procédé selon l'une des revendications 2, 3 ou 7, caractérisé en ce que l'on visualise sur l'écran commun, les unes sur les autres, les valeurs de la température et les valeurs des gradients de températures des thermogrammes, ce à l'occasion de quoi il y a un décalage réciproque optique entre le champ de températures et les points où les seuils prédéfinis de températures et/ou de gradients de températures sont dépassés.

Fig.1

Dateneingabe — 18

Speicher — 17

Rechner — 13

Anzeige-Steuerung — 14

Speicher — 12

Thermokamera — 10

A/D — 11

15 · 15a · 15b

16

1

Fig.2

Fig.3

T [°C]

29,5

29

28,5

28

27,5

5  10  15  20  25  30  35  [cm]

< 28,0°C
28,0–28,5°C
28,5–29,0°C
29,0–29,5°C
> 29,5°C

19
28
21
22
20
24
23

Fig.4

Fig.5

0 052 133